# EUROPÄISCHE PATENTSCHRIFT

(11) **EP 3 573 946 B1**
(45) Veröffentlichungstag und Bekanntmachung des Hinweises auf die Patenterteilung: **16.12.2020**
(21) Anmeldenummer: 18700600.2
(22) Anmeldetag: 22.01.2018
(51) Int. Cl.: C07C 67/04, C07C 69/54

(54) **VERFAHREN ZUR HERSTELLUNG VON (METH)ACRYLSÄURENORBORNYLESTERN**
METHOD FOR THE PREPARATION OF NORBORNYL (METH)ACRYLATE ESTERS
PROCÉDÉ DE FABRICATION D'ÉSTERS DE (MÉTH)ACRYLATE DE NORBORNYLE

(30) Priorität: 27.01.2017 EP 17153566
(43) Veröffentlichungstag der Anmeldung: 04.12.2019
(73) Patentinhaber: BASF SE, 67056 Ludwigshafen am Rhein (DE)
(72) Erfinder: MISSKE, Andrea, 67056 Ludwigshafen (DE); FLECKENSTEIN, Christoph, 67056 Ludwigshafen (DE); FLEISCHHAKER, Friederike, 67056 Ludwigshafen (DE)
(74) Vertreter: BASF IP Association
(86) Internationale Anmeldenummer: PCT/EP2018/051381
(87) Internationale Veröffentlichungsnummer: WO 2018/138025

(56) Entgegenhaltungen:
- DE-A1- 1 954 548
- M.K. MAMEDOV, ET AL.: "Synthesis of bi- and tricyclic acrylates in the presence of boron trifluoride etherate", RUSSIAN JOURNAL OF ORGANIC CHEMISTRY, Bd. 46, Nr. 5, Mai 2010 (2010-05), Seiten 628-630, XP055277455, Pleiades Publishing, Moskau, RU ISSN: 1070-4280, DOI: 10.1134/S1070428010050039 in der Anmeldung erwähnt

## Beschreibung

Die vorliegende Erfindung betrifft die Herstellung von Norbornyl-(meth)acrylat. Bei den erfindungsgemäßen Verfahren werden Norbornen und (Meth)acrylsäure in Gegenwart von Bortrifluorid umgesetzt.

Norbornyl-(meth)acrylat wird beispielsweise zur Herstellung von Copolymeren verwendet. Copolymere, die Norbornyl-(meth)acrylat als Comonomer in einpolymerisierter Form enthalten, werden beispielsweise als Bestandteil von härtbaren Zusammensetzungen verwendet. Härtbare Zusammensetzungen finden Anwendung in Klebstoffen, Dichtstoffen, Druckfarben, Drucktinten sowie in Beschichtungen für Elektronik, in Beschichtungen im Bereich der Automobilindustrie und generell im industriellen Bereich.

Verfahren zur Herstellung von Norbornyl-(meth)acrylat aus (Meth)acrylsäure und Norbornen sind dem Fachmann aus dem Stand der Technik bekannt. Im Allgemeinen erfolgt die Addition von (Meth)acrylsäure an Norbornen in Gegenwart eines sauren Katalysators. Als saure Katalysatoren eigenen sich beispielsweise Lewis-Säuren wie Bortrifluorid oder dessen Komplexe.

Im Russian Journal of Organic Chemistry (2010, Vol. 46, Seite 628-630) wird die Addition von Acrylsäure an Norbornen in Gegenwart von Bortrifluoriddiethyletherat beschrieben. Gemäß dem offenbarten Beispiel werden Norbornen, Acrylsäure und Bortrifluoriddiethyletherat vorgelegt und erhitzt. Norbornyl-(meth)acrylat wird anschließend durch fraktionierte Destillation mit einer Ausbeute von 80% aus der Reaktionsmischung isoliert.

DE 19 54 548 A1 (BASF AG) offenbart ein Verfahren zur Herstellung von Dihydrodicyclopentadienylacrylat durch die Zugabe von Dicyclopentadien zu einer Mischung aus Acrylsäure, Hydrochinon und Bortrifluorid-Dimethyletherat bei einer Temperatur zwischen 70 und 80° C (Beispiel 1). Das Produkt wird durch Auswaschung mit Wasser isoliert.

Aufgabe war es, ein verbessertes Verfahren zur Herstellung von Norbornyl-(meth)acrylat zur Verfügung zu stellen. Durch das verbesserte Verfahren sollte es möglich sein, Norbornyl-(meth)acrylat in höherer Selektivität, Ausbeute und Reinheit herzustellen. Des Weiteren war es wünschenswert, wenn Norbornyl-(meth)acrylat mit nur geringem Aufwand in hoher Reinheit und Ausbeute isoliert werden kann. So ist es beispielsweise vorteilhaft, wenn Norbornyl-(meth)acrylat ohne fraktionierte Destillation aus der Reaktionsmischung in hoher Reinheit und Ausbeute isoliert werden kann.

Die Aufgabe wird gelöst durch ein Verfahren zur Herstellung von Norbornyl-(meth)acrylat durch Umsetzung von Norbornen mit (Meth)acrylsäure in Gegenwart von Bortrifluorid als Katalysator, das dadurch gekennzeichnet ist, dass man
a) Bortrifluorid in (Meth)acrylsäure vorlegt,
b) die Vorlage auf eine Temperatur von 75 bis 110° C erwärmt,
c) Norbornen zugibt
   und
d) das erhaltene Norbornyl-(meth)acrylat aus der Reaktionsmischung isoliert.
Die Aufgabe wird auch gelöst durch ein Verfahren zur Herstellung von Norbornyl-(meth)acrylat durch Umsetzung von Norbornen mit (Meth)acrylsäure in Gegenwart von Bortrifluorid als Katalysator, das dadurch gekennzeichnet ist, dass man
a) Bortrifluorid in einem organischen Lösungsmittel vorlegt,
b) die Vorlage auf eine Temperatur von 75 bis 110° C erwärmt,
c) eine Mischung enthaltend Norbornen und (Meth)acrylsäure zugibt und
d) das erhaltene Norbornyl-(meth)acrylat aus der Reaktionsmischung isoliert.

Norbornyl-(meth)acrylat ist Norbornylacrylat oder Norbornylmethacrylat.

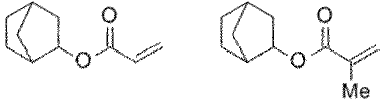

(Meth)acrylsäure ist Acrylsäure oder Methacrylsäure. Die in den erfindungsgemäßen Verfahren eingesetzte (Meth)acrylsäure ist in der Regel möglichst rein. Möglichst reine (Meth)acrylsäure weist eine Reinheit von mindestens 95 Gewichtsprozent auf. Es ist bevorzugt, dass die in den erfindungsgemäßen Verfahren eingesetzte (Meth)acrylsäure eine Reinheit von mindestens 97 Gewichtsprozent und weiter bevorzugt eine Reinheit von mindestens 99 Gewichtsprozent aufweist.

In den erfindungsgemäßen Verfahren wird (Meth)acrylsäure in einer Menge von 100 bis 1000 Molprozent, bevorzugt 105 bis 750 Molprozent und besonders bevorzugt 110 bis 250 Molprozent, bezogen auf die Menge an Norbornen eingesetzt.

Auch wenn (Meth)acrylsäure ohne Stabilisator in den erfindungsgemäßen Verfahren eingesetzt werden kann, ist es in der Regel bevorzugt, dass die eingesetzte (Meth)acrylsäure einen Stabilisator oder eine Mischung verschiedener Stabilisatoren enthält.
Ein Stabilisator oder eine Mischung verschiedener Stabilisatoren dient im Allgemeinen dazu, die Polymerisation von (Meth)acrylsäure oder der korrespondierenden Ester zu inhibieren.

Die Menge an Stabilisator, die in der eingesetzten (Meth)acrylsäure enthalten ist, richtet sich im Allgemeinen nach der Art des verwendeten Stabilisators, beziehungsweise nach der Art der verwendeten Stabilisatoren, wenn eine Mischung verschiedener Stabilisatoren verwendet wird. In der Regel beträgt die Menge an Stabilisator, die in der eingesetzten (Meth)acrylsäure enthalten ist, 50 bis 1000 ppm, bevorzugt 100 bis 800 ppm und weiter bevorzugt 150 bis 300 ppm.

Stabilisatoren, die die Polymerisation von (Meth)acrylsäure inhibieren sind dem Fachmann bekannt oder erschließen sich ihm aus seinem allgemeinen Fachwissen. Bekannte Stabilisatoren sind beispielsweise Kupfer(meth)acrylate, Kupferdithiocarbamate, Phenothiazine, phenolische Verbindungen, N-Oxyle, Phenylendiamine, Nitrosoverbindungen, Harnstoffe oder Thioharnstoffe. Diese Stabilisatoren können einzeln oder als beliebige Mischung eingesetzt werden. Bevorzugte Stabilisatoren sind Phenothiazine, phenolische Verbindungen, N-Oxyle oder beliebige Mischungen dieser.

Phenothiazine können beispielsweise Phenothiazin, Bis-(*α*-methylbenzyl)phenothiazin, 3,7-Dioctylphenothiazin, Bis-(*α*-dimethylbenzyl)phenothiazin oder eine beliebige Mischung dieser sein.

Phenolische Verbindungen können beispielsweise Hydrochinon, Hydrochinonmonomethylether, wie para-Methoxyphenol (MEHQ), Pyrogallol, Catechol, Resorcin, Phenol, Kresol, 2,4-Dimethyl-6-tert-butylphenol, 2,6-Di-tert-butyl-para-kresol oder eine beliebige Mischung dieser sein. Bevorzugte phenolische Verbindungen sind para-Methoxyphenol (MEHQ), 2,4-Dimethyl-6-tert-butylphenol, 2,6-Di-tert-butyl-para-kresol oder eine beliebige Mischung dieser. Para-Methoxyphenol (MEHQ) ist besonders bevorzugt.

N-Oxyle können beispielsweise Di-tert-butylnitroxid, 2,2,6,6-Tetramethyl-4-hydroxypiperidyl-1-oxyl, 2,2,6,6-Tetramethylpiperidyl-1-oxyl, 2,2,6,6-Tetramethylpiperidinoxyl, 4-Hydroxy-2,2,6,6-tetramethylpiperidinoxyl, 4,4',4"-Tris-1-(2,2,6,6-tetramethylpiperidinoxyl)phosphite oder eine beliebige Mischung dieser sein.

Es ist besonders bevorzugt, dass die in den erfindungsgemäßen Verfahren eingesetzte (Meth)acrylsäure mit para-Methoxyphenol (MEHQ) stabilisiert ist. Die Menge an MEHQ in der in den erfindungsgemäßen Verfahren eingesetzten (Meth)acrylsäure beträgt bevorzugt 150 bis 300 ppm.

Norbornen wird in möglichst hoher Reinheit in den erfindungsgemäßen Verfahren eingesetzt. Im Allgemeinen wird Norbornen mit einer Reinheit von mindestens 90 Gewichtsprozent eingesetzt. Es ist bevorzugt, dass Norbornen mit einer Reinheit von mindestens 95 Gewichtsprozent und weiter bevorzugt mit einer Reinheit von mindestens 97 Gewichtsprozent eingesetzt wird. Die Gewinnung von Norbornen in entsprechender Reinheit ist dem Fachmann bekannt oder erschließt sich ihm aus seinem allgemeinen Fachwissen.

Nach den erfindungsgemäßen Verfahren wird Bortrifluorid in Schritt a) in (Meth)acrylsäure oder in einem organischen Lösungsmittel vorgelegt. Im Allgemeinen liegen (Meth)acrylsäure oder das organische Lösungsmittel in der flüssigen Phase vor und Bortrifluorid wird der vorliegenden flüssigen Phase zugegeben. Es ist bevorzugt, dass Bortrifluorid in (Meth)acrylsäure oder in einem organischen Lösungsmittel bei einer Temperatur von 15 bis 50° C vorgelegt wird. Weiter ist es bevorzugt, dass Bortrifluorid in (Meth)acrylsäure oder in einem organischen Lösungsmittel bei einer Temperatur von 20 bis 35° C vorgelegt wird.

Bortrifluorid wird bevorzugt in Gegenwart von Sauerstoff in (Meth)acrylsäure oder in einem organischen Lösungsmittel vorgelegt. So kann Bortrifluorid beispielsweise in Gegenwart eines sauerstoffhaltigen Gases wie Luft, Magerluft oder getrockneter Luft in (Meth)acrylsäure oder in einem organischen Lösungsmittel vorgelegt werden. Im Rahmen der erfindungsgemäßen Verfahren erweist sich die Gegenwart von Sauerstoff generell als vorteilhaft, da dadurch die Inhibierung der Polymerisation der eingesetzten (Meth)acrylsäure und/oder des hergestellten Norbornyl-(meth)acrylats positiv beeinflusst wird.

Im Allgemeinen wird Bortrifluorid gasförmig oder als Komplex der vorgelegten (Meth)acrylsäure oder dem vorgelegten organischen Lösungsmittel zugeführt. Wird Bortrifluorid gasförmig zugeführt, ist es von Vorteil, wenn Bortrifluorid in die vorgelegte (Meth)acrylsäure oder in das vorgelegte organische Lösungsmittel eingeleitet wird. Die Einleitung kann beispielsweise durch ein oder mehrere gegenüber Bortrifluorid korrosionsbeständige Tauchrohre oder Düsen erfolgen. Durch Einleitung von gasförmigem Bortrifluorid in die vorgelegte (Meth)acrylsäure oder in das vorgelegt organische Lösungsmittel kann es zur Komplexbildung kommen. Wird Bortrifluorid als Komplex der vorgelegten (Meth)acrylsäure oder dem vorgelegten organischen Lösungsmittel zugeführt, kann es zu einer Umkomplexierung kommen.

Komplexe von Bortrifluorid sind beispielsweise Bortrifluorid-Etherate, Bortrifluorid-Acetonitril-Komplexe, Bortrifluorid-Hydrate, Bortrifluorid-Carbonsäure-Komplexe wie Bortrifluorid-Essigsäure-Komplexe oder Bortrifluorid-(Meth)acrylsäurekomplexe oder beliebige Mischungen aus diesen Komplexen. Bortrifluorid-Etherate sind bevorzugt. Bortrifluorid-Etherate können beispielsweise Bortrifluorid-Dimethyletherat, Bortrifluorid-Diethyletherat, Bortrifluorid-Tetrahydrofuran-Komplexe oder beliebige Mischungen dieser sein. Unter den Bortrifluorid-Etheraten sind Bortri-fluorid-Dimethyletherat und/oder Bortrifluorid-Diethyletherat bevorzugt. Bortrifluorid-Dimethyletherat ist besonders bevorzugt.

Wird Bortrifluorid als Komplex der vorgelegten (Meth)acrylsäure oder dem vorgelegten organischen Lösungsmittel zugeführt, kann es sein, dass der Komplex in einem Lösungsmittel gelöst ist. Meist handelt es sich bei dem Lösungsmittel um diejenige Verbindung, die zur Komplexbildung eingesetzt wurde.

In den erfindungsgemäßen Verfahren wird Bortrifluorid in einer Menge von 0,1 bis 5 Molprozent, bevorzugt in einer Menge von 0,2 bis 2,5 Molprozent und weiter bevorzugt in einer Menge von 0,5 bis 1,5 Molprozent bezogen auf die Menge an Norbornen eingesetzt. So kann Bortrifluorid beispielsweise in einer Menge von 0,6, 0,7, 0,8, 0,9, 1,0, 1,1, 1,2, 1,3 oder 1,4 Molprozent bezogen auf die Menge an Norbornen eingesetzt werden.

Als organisches Lösungsmittel, in dem Bortrifluorid vorgelegt wird, eignen sich prinzipiell alle organischen Lösungsmittel, die keine unerwünschten Reaktionen mit den Edukten, dem Katalysator und den Produkten eingehen und zur unerwünschten Nebenproduktbildung führen. Es ist vorteilhaft, wenn der Siedepunkt des organischen Lösungsmittels über 75° C bei Normaldruck liegt.

Als organische Lösungsmittel können polar aprotische Lösungsmittel, unpolar aprotische Lösungsmittel, (Meth)acrylsäure, Norbornyl-(meth)acrylat oder eine beliebige Mischung dieser verwendet werden. Polar aprotische Lösungsmittel können beispielsweise Nitrile, wie Acetonitril, Nitroverbindungen, wie Nitromethan, Sulfoxide, wie Dimethylsulfoxid, Lactame, wie N-Methyl-2-pyrrolidon, tertiäre Carbonsäureamide, wie Dimethylformamid, Ketone, Dichlormethan, Tri-chlormethan oder eine beliebige Mischung dieser sein. Unpolar aprotische Lösungsmittel können beispielsweise Ether, wie Alkylether, Toluol oder eine beliebige Mischung dieser sein. Alkylether können beispielsweise Dimethylether, Diethylether, Methyl-tert-butylether, Tetrahydrofuran, 1,4-Dioxan oder eine beliebige Mischung dieser sein. Die Verwendung von Norbornen als organisches Lösungsmittel hat sich nicht als vorteilhaft herausgestellt. Bevorzugte organische Lösungsmittel sind (Meth)acrylsäure, Norbornyl-(meth)acrylat, Ether, wobei 1,4-Dioxan bevorzugt ist, oder eine beliebige Mischung dieser.

Der vorgelegten (Meth)acrylsäure oder dem vorgelegten organischen Lösungsmittel kann zusätzlich ein Stabilisator oder eine Mischung verschiedener Stabilisatoren zugeführt werden. Wird der vorgelegten (Meth)acrylsäure oder dem vorgelegten organischen Lösungsmittel ein Stabilisator oder eine Mischung verschiedener Stabilisatoren zusätzlich zugeführt, erfolgt dies in der Regel bevor Bortrifluorid der vorgelegten (Meth)acrylsäure oder dem vorgelegten organischen Lösungsmittel zugeführt wird.

Die Menge an Stabilisator, die der vorgelegten (Meth)acrylsäure oder dem vorgelegten organischen Lösungsmittel zusätzlich zugeführt wird, richtet sich nach der Art des Stabilisators beziehungsweise nach der Art der Mischung verschiedener Stabilisatoren. In der Regel beträgt die Menge an Stabilisator, die zusätzlich zugeführt wird 0,005 bis 0,15 und bevorzugt 0,05 bis 0,15 Molprozent, bezogen auf die Menge, der in den erfindungsgemäßen Verfahren eingesetzten (Meth)acrylsäure.

Als Stabilisator können die oben genannten Stabilisatoren verwendet werden. In der Regel ist es von Vorteil, den Stabilisator oder die Mischung verschiedener Stabilisatoren zu verwenden, die in der, in den erfindungsgemäßen Verfahren eingesetzten, (Meth)acrylsäure bereits enthalten sind. Demnach ist es bevorzugt, dass MEHQ der vorgelegten (Meth)acrylsäure oder dem vorgelegten organischen Lösungsmittel zusätzlich zugeführt wird.

In bevorzugten Verfahrensausgestaltungen wird Bortrifluorid in (Meth)acrylsäure, Norbornyl-(meth)acrylat oder in 1,4-Dioxan vorgelegt. Bortrifluorid wird dabei gasförmig oder als Komplex, bevorzugt als Dimethyletherat oder als Diethyletherat vorgelegt. Die Temperatur bei der Bortrifluorid in (Meth)acrylsäure, Norbornyl-(meth)acrylat oder in 1,4-Dioxan vorgelegt wird beträgt dabei 15 bis 50° C. Die Temperatur kann beispielsweise 18, 20, 22, 24, 26, 28, 30, 32 oder 35 ° C betragen.
In weiter bevorzugten Verfahrensausgestaltungen wird Bortrifluorid in (Meth)acrylsäure, Norbornyl-(meth)acrylat oder in 1,4-Dioxan vorgelegt. Bortrifluorid wird dabei gasförmig oder als Komplex, bevorzugt als Dimethyletherat oder als Diethyletherat, in einer Menge von 0,5 bis 1,5 Molprozent bezogen auf die Menge an Norbornen vorgelegt. Die Temperatur bei der Bortrifluorid in (Meth)acrylsäure, Norbornyl-(meth)acrylat oder in 1,4-Dioxan vorgelegt wird, beträgt dabei 15 bis 50° C. Die Temperatur kann beispielsweise 18, 20, 22, 24, 26, 28, 30, 32 oder 35 ° C betragen.

Die erfindungsgemäßen Verfahren können in dem Fachmann bekannten Reaktoren ausgeführt werden. Als Reaktoren können beispielsweise Rührkesselreaktoren, Schlaufenreaktoren, Rohrreaktoren oder beliebige Kombinationen dieser verwendet werden. Die Reaktoren sind in der Regel aus metallischem Material, wobei Edelstahl bevorzugt ist.

Bei den Reaktoren kann es sich um Reaktoren mit Doppelwandheizung und/oder innenliegenden Heizschlangen handeln. Auch kann es sich bei den Reaktoren um Reaktoren mit außenliegenden Wärmetauschern und Natur- oder Zwangsumlauf (unter Verwendung einer Pumpe) handeln. Die Durchmischung in den Reaktoren kann durch Rührvorrichtungen und/oder durch Einspeisung eines Gases, vorzugsweise eines sauerstoffhaltigen Gases erfolgen.

Die erfindungsgemäßen Verfahren können kontinuierlich oder diskontinuierlich ausgeführt werden, wobei eine diskontinuierliche Ausführung bevorzugt ist. Im Allgemeinen sind Reaktoren, die sich für eine diskontinuierliche Reaktionsführung eignen, beispielsweise Rührkesselreaktoren, bevorzugt. Mehrere Reaktoren, bevorzugt Rührkesselreaktoren, können in Reihe und/oder parallel geschaltet werden. Bei der Auswahl geeigneter Reaktoren und deren Verschaltung lässt sich der Fachmann von seinem allgemeinen Fachwissen und von praktischen Überlegungen leiten.

In Schritt b) der erfindungsgemäßen Verfahren wird die Vorlage erwärmt. Die Vorlage wird auf eine Temperatur von 75 bis 110° C, bevorzugt 80 bis 105° C und weiter bevorzugt auf eine Temperatur von 85 bis 100° C erwärmt.

Das Erwärmen der Vorlage erfolgt in der Regel in Gegenwart von Sauerstoff. So können beispielsweise sauerstoffhaltige Gase wie Luft, Magerluft oder getrocknete Luft der Vorlage während dem Erwärmen zugeführt werden. Das sauerstoffhaltige Gas, das der Vorlage während des Erwärmens zugeführt wird, wird im Allgemeinen dem Gasraum über der Vorlage zugeführt. Das sauerstoffhaltige Gas kann aber auch direkt in die Vorlage, beispielsweise durch ein oder mehrere Tauchrohre oder Düsen, durch die das sauerstoffhaltige Gas ausströmt, zugeführt werden.

In Schritt c) der erfindungsgemäßen Verfahren wird Norbornen oder eine Mischung enthaltend Norbornen und (Meth)acrylsäure der erwärmten Vorlage zugegeben. Wird Norbornen oder eine Mischung enthalten Norbornen und (Meth)acrylsäure zugegeben, hat die Vorlage eine Temperatur von 75 bis 110° C, bevorzugt 80 bis 105° C und weiter bevorzugt eine Temperatur von 85 bis 100° C.

Norbornen oder eine Mischung enthaltend Norbornen und (Meth)acrylsäure kann der erwärmten Vorlage einmalig oder schrittweise zugegeben werden. Die schrittweise Zugabe kann kontinuierlich oder diskontinuierlich erfolgen. Im Allgemeinen wird Norbornen oder eine Mischung enthalten Norbornen und (Meth)acrylsäure kontinuierlich der erwärmten Vorlage zugegeben.

Die Geschwindigkeit der Zugabe richtet sich nach der Wärmeentwicklung der Reaktion und ist dabei so zu wählen, dass die Temperatur der Vorlage nicht mehr als 20° C, bevorzugt nicht mehr als 10° C und besonders bevorzugt nicht mehr als 5° C ansteigt.

Eine kontinuierliche Zugabe von Norbornen oder einer Mischung enthaltend Norbornen und (Meth)acrylsäure kann durch, dem Fachmann bekannte, Fördermittel erfolgen. So kann eine kontinuierliche Zugabe beispielsweise über eine Schnecke, ein Förderband, eine Pumpe, eine Fördertrommel oder eine beliebige Kombination dieser erfolgen.

Norbornen kann in fester oder flüssiger Form der erwärmten Vorlage zugegeben werden. Die Auswahl eines geeigneten Fördermittels passt der Fachmann selbstverständlich der Beschaffenheit des zu fördernden Stoffes, beziehungsweise Stoffgemisches an. Es ist bevorzugt, dass Norbornen in flüssiger Form der erwärmten Vorlage zugegeben wird. Festes Norbornen wird hierzu aufgeschmolzen. Ein Teil der dafür benötigen Wärme kann beispielsweise durch Wärmeintegration mit der erwärmten Vorlage, beispielsweise durch Abwärme der erwärmten Vorlage, gewonnen werden. Über beheizte Leitungen und/oder Fördermittel kann das flüssige Norbornen dann der erwärmten Vorlage zugegeben werden.

Eine Mischung enthaltend Norbornen und (Meth)acrylsäure wird im Allgemeinen in flüssiger Form der erwärmten Vorlage zugegeben. Dazu kann Norbornen in fester oder flüssiger Form in flüssiger (Meth)acrylsäure gelöst werden. Neben Norbornen und (Meth)acrylsäure kann die Mischung auch einen oder eine Mischung verschiedener Stabilisatoren enthalten. Auch wenn in der Regel (Meth)acrylsäure eingesetzt wird, die bereits einen oder eine Mischung verschiedener Stabilisatoren enthält, ist es von Vorteil der Mischung zusätzlich einen Stabilisator oder Mischung verschiedener Stabilisatoren zuzuführen. Wird ein Stabilisator oder eine Mischung verschiedener Stabilisatoren der Mischung, enthaltend Norbornen und (Meth)acrylsäure, zusätzlich zugeführt, ist es von Vorteil, einen Stabilisator oder eine Mischung von verschiedenen Stabilisatoren zu verwenden, die bereits in der (Meth)acrylsäure enthalten sind.

Die Menge an Stabilisator, die der Mischung, enthaltend Norbornen und (Meth)acrylsäure, zusätzlich zugeführt wird, richtet sich nach der Art des Stabilisators, beziehungsweise nach der Art der Mischung verschiedener Stabilisatoren. In der Regel beträgt die Menge an Stabilisator, die der Mischung, enthaltend Norbornen und (Meth)acrylsäure, zusätzlich zugeführt wird 0,005 bis 0,15 und bevorzugt 0,05 bis 0,15 Molprozent, bezogen auf die Menge der in der Mischung enthaltenen (Meth)acrylsäure.

Als Stabilisator können die oben genannten Stabilisatoren verwendet werden, wobei MEHQ bevorzugt ist.

Die Zugabe von Norbornen oder einer Mischung enthaltend Norbornen und (Meth)acrylsäure zur erwärmten Vorlage erfolgt vorteilhafterweise in Gegenwart von Sauerstoff. Ein sauerstoffhaltiges Gas kann dazu in die erwärmte Vorlage, in den Gasraum über der erwärmten Vorlage, in die Zugabeleitungen und/oder in den Zugabestrom von Norbornen oder einer Mischung enthaltend Norbornen und (Meth)acrylsäure zugeführt werden. Wird das sauerstoffhaltige Gas in die erwärmte Vorlage oder in den Zugabestrom von Norbornen oder einer Mischung enthaltend Norbornen und (Meth)acrylsäure zugeführt, kann dies beispielsweise über ein oder mehrere Tauchrohre oder Düsen erfolgen, durch die das sauerstoffhaltige Gas ausströmt.

Nach Umsetzung der Unterschusskomponente, in der Regel Norbornen, wird das erhaltene Norbornyl-(meth)acrylat aus der Reaktionsmischung in Schritt d) der erfindungsgemäßen Verfahren isoliert. Bei der Reaktionsmischung handelt es sich um die Mischung, die nach den Schritten a) bis c) der erfindungsgemäßen Verfahren erhalten wird.

Norbornyl-(meth)acrylat kann aus der Reaktionsmischung durch Extraktion und/oder destillative Abtrennung (Destillation) der leichter siedenden Verbindungen isoliert werden.

Wird Norbornyl-(meth)acrylat durch Extraktion isoliert, kann dies durch einen oder mehrere Extraktionsschritte erfolgen.

Im Allgemeinen wird die Reaktionsmischung hierzu mit einer wässrigen Lösung einer Base versetzt und die Phasen anschließend getrennt. Die Konzentration der wässrigen Basenlösung entspricht den üblichen Konzentration, die zu Extraktionszwecken verwendet werden und kann über einen weiten Bereich variieren. Vorteilhafte Konzentrationen können vom Fachmann durch wenige Routineversuche bestimmt werden, oder erschließen sich ihm aus seinem allgemeinen Fachwissen oder anhand von praktischen Überlegungen.

Bei einer Base handelt es sich beispielsweise um Natriumhydroxid, Kaliumhydroxid, Natriumhydrogencarbonat, Kaliumhydrogencarbonat, Calciumhydroxid, Ammoniak, Kaliumcarbonat, oder beliebige Mischungen dieser. Es ist bevorzugt, dass es sich bei der Base um Natriumhydroxid handelt. Die wässrige Lösung einer Base kann zusätzlich ein weiteres Salz enthalten. Bei einem weiteren Salz handelt es sich beispielsweise um Natriumchlorid, Kaliumchlorid, Ammoniumchlorid, Ammoniumsulfat, oder um beliebige Mischungen dieser. Es ist bevorzugt, dass es sich bei dem weiteren Salz um Natriumchlorid handelt. Die Menge des weiteren Salzes entspricht den üblichen Mengen, die zu Extraktionszwecken verwendet lassen. Vorteilhafte Mengen können vom Fachmann durch wenige Routineversuche bestimmt werden, oder erschließen sich ihm aus seinem allgemeinen Fachwissen oder anhand von praktischen Überlegungen.

Die Zugabe der wässrigen Lösung einer Base erfolgt in einer Weise, dass die Temperatur der Reaktionsmischung nicht über 40° C ansteigt und der pH-Wert nach Zugabe der wässrigen Lösung einer Base 10 bis 14 beträgt. Die Abfuhr der Neutralisationswärme erfolgt gegebenenfalls durch Kühlung der Reaktionsmischung, beispielsweise durch innenliegende Kühlschlangen oder über eine Doppelwandkühlung. Der Behälter in diesem die Reaktionsmischung mit einer wässrigen Lösung einer Base versetzt wird, ist demnach entsprechend auszugestalten.

Zusätzlich kann ein organisches Lösungsmittel, das schlecht in Wasser löslich ist, der Reaktionsmischung zugesetzt werden. Dies kann beispielsweise dazu dienen, die Temperaturentwicklung bei der Zugabe einer wässrigen Lösung einer Base besser zu kontrollieren. Ein organisches Lösungsmittel, das schlecht in Wasser löslich ist, hat eine Löslichkeit in Wasser von weniger als 10g/l Wasser bei 20° C, bevorzugt weniger als 1g/l Wasser bei 20° C.

Das Mengenverhältnis Reaktionsmischung : wässrige Lösung einer Base kann über weite Bereiche variieren. Vorteilhafte Mengenverhältnisse können vom Fachmann durch wenige Routineversuche bestimmt werden, oder erschließen sich ihm aus seinem allgemeinen Fachwissen oder anhand von praktischen Überlegungen.

Zur Entfernung von Base- und/oder Salzspuren aus der Reaktionsmischung nach der Phasentrennung kann eine Nachwäsche vorteilhaft sein. Hierzu kann die Reaktionsmischung mit einer Waschflüssigkeit behandelt werden. Bei einer Waschflüssigkeit handelt es sich beispielsweise um Wasser. Es kann von Vorteil sein, dass Wasser als Waschflüssigkeit ein Salz enthält. Bei einem Salz handelt es sich beispielsweise um Natriumchlorid, Kaliumchlorid, Ammoniumchlorid, Natriumsulfat, Aluminiumsulfat oder um beliebige Mischungen dieser. Die Menge an Salz kann über weite Bereiche variieren. Vorteilhafte Mengen können vom Fachmann durch wenige Routineversuche bestimmt werden, oder erschließen sich ihm aus seinem allgemeinen Fachwissen oder anhand von praktischen Überlegungen.

Verfahrenstechnisch können für eine Extraktion in den erfindungsgemäßen Verfahren alle an sich bekannten Extraktions- und Waschverfahren und -apparate eingesetzt werden, beispielsweise solche, die in Ullmann's Encyclopedia of Industrial Chemistry, 6th Ed., 1999 Electronic Release, Kapitel "Liquid - Liquid Extraction - Apparatus" beschrieben sind. Beispielsweise können dies ein- oder mehrstufige, bevorzugt einstufige Extraktionen sowie Extraktionen in Gleich- oder Gegenstromfahrweise sein. Behälter die sich für die Extraktion eignen sind beispielsweise Rührbehälter, Kolonnen oder Mixer-Settler-Apparaturen.

Die nach der Extraktion erhaltene organische Phase, die Norbornyl-(meth)acrylat enthält, wird gegebenenfalls mit einem Stabilisator oder einer Mischung verschiedener Stabilisatoren versetzt um eine vorteilhafte Stabilisatorkonzentration einzustellen. Die einzustellende Stabilisatorkonzentration hängt im Allgemeinen von der jeweiligen Spezifikation des Endproduktes ab und liegt für kommerziell erhältliche Alkyl-(meth)acrylate im Bereich von 15 bis 200 ppm. So kann es vorteilhaft sein, eine Stabilisatorkonzentration von 30, 50, 80, 100, 120, 150 oder 180 ppm einzustellen.

Als Stabilisatoren werden in der Regel phenolische Verbindungen, wie 2,6-Di-tert.-Butyl-4-methylphenol, 6-tert.-Butyl-2,4-dimethylphenol, Hydrochinon, Hydrochinonmonomethylether oder eine beliebige Mischung dieser verwendet. Hydrochinonmonomethylether (MEHQ) ist bevorzugt.

Nach der Extraktion kann die organische Phase, die Norbornyl-(meth)acrylat und gegebenenfalls einen Stabilisator oder eine Mischung verschiedener Stabilisatoren enthält nach dem Fachmann bekannten Verfahren weiter aufgearbeitet werden, um Norbornyl-(meth)acrylat zu erhalten. Hierzu kann Norbornyl-(meth)acrylat, gegebenenfalls zusammen mit dem Stabilisator oder Mischung verschiedener Stabilisatoren, beispielsweise durch Destillation oder Strippung von den anderen Bestandteilen der organischen Phase abgetrennt werden. Die abgetrennten Bestandteile der organischen Phase, vorzugsweise das organische Lösungsmittel können beispielsweise wieder in der Extraktion verwendet werden.

Wird Norbornyl-(meth)acrylat durch destillative Abtrennung der leichter siedenden Verbindungen isoliert, kann dies in einem oder in mehreren Destillationsschritten erfolgen. Im Allgemeinen werden nicht umgesetzte (Meth)acrylsäure, gegebenenfalls das organische Lösungsmittel, Bortrifluorid und andere leichter flüchtige Verbindungen destillativ aus der Reaktionsmischung abgetrennt. Norbornyl-(meth)acrylat bleibt als Sumpffraktion zurück. Durch die erfindungsgemäßen Verfahren wird die Bildung von höher siedenden Nebenprodukten, beispielsweise Nebenprodukte, die durch Dimerisierung von (Meth)acrylsäure entstehen, minimiert, wodurch auf eine fraktionierte Destillation des Norbornyl-(meth)acrylats verzichtet werden kann.

Da Norbornyl-(meth)acrylat selbst nicht destillativ isoliert werden muss und als Sumpffraktion in hoher Reinheit anfällt, kann die Destillation der leichter siedenden Verbindungen in einfachen Apparaturen erfolgen.

Zur destillativen Abtrennung der leichter siedenden Verbindungen eignen sich in der Regel alle Apparaturen zur destillativen Auftrennung von Reaktionsgemischen, die flüssige Komponenten enthalten. Geeignete Apparaturen umfassen Destillationskolonnen, wie Bodenkolonnen, die mit Glockenböden, Siebplatten, Siebböden, Packungen oder Füllkörpern ausgerüstet sein können, oder Drehbandkolonnen-Verdampfer, wie Dünnschichtverdampfer, Fallfilmverdampfer, Zwangsumlaufverdampfer, Sambay-Verdampfer, etc. und Kombinationen davon.
Es können ein oder mehrere Destillationsschritte hintereinandergeschaltet sein. Die Destillationsschritte können in gleichen oder in verschiedenen Apparaturen erfolgen.

Bei der Auswahl geeigneter Temperatur- und Druckbereiche zur destillativen Abtrennung der leichter siedenden Verbindungen lässt sich der Fachmann von den physikalischen Gegebenheiten der Trennaufgabe (beispielsweise Dampfdruckkurven) sowie von seinem allgemeinen Fachwissen und von praktischen Überlegungen leiten.

Es ist möglich Extraktion und Destillation zu kombinieren. So kann die Reaktionsmischung beispielsweise zuerst einer Extraktion und anschließend einer Destillation unterzogen werden, oder umgekehrt. Bei einer Kombination von Extraktion und Destillation ist es generell bevorzugt die Reaktionsmischung zuerst einer Destillation zu unterziehen, um die leichter siedenden Verbindungen abzutrennen. Die nach Abtrennung der leichter siedenden Verbindungen erhaltene Sumpffraktion, die Norbornyl-(meth)acrylat enthält, kann dann durch Extraktion weiter aufgearbeitet werden.

Durch die erfindungsgemäßen Verfahren ist es möglich Norbornyl-(meth)acrylat in hoher Reinheit und in hoher Ausbeute darzustellen. So kann Norbornyl-(meth)acrylat in einer Reinheit von mindestens 99 Gewichtsprozent isoliert werden. Die Ausbeute an Norbornyl-(meth)acrylat beträgt dabei mindestens 90 Prozent.

Vorteilhafterweise muss Norbornyl-(meth)acrylat selbst keiner destillativen oder rektifikativen Aufreinigung unterzogen werden, um die hohe Reinheit zu erreichen. Wie bereits ausgeführt kann Norbornyl-(meth)acrylat in hoher Reinheit durch Extraktion und/oder destillativer Abtrennung der leichter siedenden Verbindungen isoliert werden. Dies hat den weiteren Vorteil, dass die Herstellung und Isolierung von Norbornyl-(meth)acrylat in relativ simplen Apparaturen erfolgen kann. Auch wird die thermische Belastung von Norbornyl-(meth)acrylat bei der Isolierung reduziert, wodurch die Bildung von Nebenprodukten minimiert werden kann.

Um die Farbzahl des nach den erfindungsgemäßen Verfahren hergestellten Norbornyl-(meth)acrylats zu reduzieren kann es von Vorteil sein, dass Norbornyl-(meth)acrylat destilliert wird. Hierzu kann Norbornyl-(meth)acrylat beispielsweise direkt durch Destillation aus der Reaktionsmischung isoliert werden. Wurde Norbornyl-(meth)acrylat durch Extraktion und/oder destillative Abtrennung der leichter siedenden Verbindungen isoliert, kann auch das so erhaltene Norbornyl-(meth)acrylat einer Destillation unterzogen werden.

Das durch die erfindungsgemäßen Verfahren hergestellte Norbornyl-(meth)acrylat eignet sich aufgrund seiner hohen Reinheit besonders für die Herstellung von Homo- oder Copolymeren.

Copolymere, die Norbornyl-(meth)acrylat in einpolymerisierter Form enthalten eigenen sich als Bestandteil von härtbaren Zusammensetzungen. Solche härtbaren Zusammensetzungen eigenen sich beispielsweise für die Verwendung in Haftklebstoffen, Druckfarben, insbesondere Siebdruckfarben, Drucktinten sowie für Lacke, insbesondere für Grundierungen, Decklacke, Basislacke oder Klarlacke. Auch eignen sich solche härtbaren Zusammensetzungen für die Verwendungen in Beschichtungen für LCD- und LED-Displays, in Beschichtungen für Glasflaschen, insbesondere Bierflaschen, in Beschichtungen für Kunststoffflaschen, insbesondere Shampooflaschen, in Beschichtungen für Thermopapier und in Beschichtungen für reflektierende Folien.

Solange nichts Gegenteiliges angegeben ist, beziehen sich alle Angaben zu ppm auf das jeweilige Gesamtgewicht.

Alle Angaben zu Gewichtsprozent (Gew.%) beziehen sich, solange nichts Gegenteiliges angegeben ist, auf das jeweilige Gesamtgewicht.

Die Bestimmung der Reinheit des Norbornyl-(meth)acrylats erfolgte durch Gaschromatographie wie im Experimentalteil angegeben.

### Experimentalteil:

Die Reinheit wurde mittels Gaschromatographie bestimmt. Als Lösemittel für die Proben wurde Dichlormethan der Fa. Aldrich verwendet, Reinheit 99,8%.
Als Gerät wurde ein Gaschromatograph der Firma Hewlett Packard (7890B) mit FID-Detektor & Säule 50m CP-Sil 5 CB 50m ^{∗} 0,25mm ^{∗} 0,25µm der Firma Agilent benutzt.
Als Temperaturprogramm wurde eingestellt: 60° C Start, dann mit 15° C/min auf 280° C, 1 min bei 280° C, Gesamtlaufzeit 15,7 min.

Die Hazen Farbzahl wurde mit einem Farbzahlmessgerät der Fa. Hach Lange (LICO 620) gemessen und für Normlichtart C und 2° -Normalbeobachter entsprechend DIN 5033 berechnet.

### Verwendete Edukte:

| | Quelle | Reinheit | Stabilisierung |
|---|---|---|---|
| Methacrylsäure, rein | BASF | >99.5% | 200 ± 20 ppm MEHQ |
| Acrylsäure, rein | BASF | >99.5% | 200 ± 20 ppm MEHQ |
| Norbornen | Aldrich | >99% | |
| BF3 Dimethyl-Etherat | Aldrich | 59-61 %BF3 | |
| MEHQ | Aldrich | >99% | |
| Dioxan | Aldrich | >99.5% | |
| DCM | BASF | >98% | |
| NaOH | BerndKraft | reinst | |
| NaCl | BerndKraft | >99% | |

### Beispiel 1: Norbornylacrylat unter Vorlage von Norbornylacrylat:

In einem 250 mL Vierhalsrundkolben mit Rückflusskühler, Magnetrührer, Thermometer, Tropftrichter und Lufteinleitung wurden 5 mL Norbornylacrylat vorgelegt. 0,5 ml Bortrifluoriddimethyletherat wurden zugegeben. Eine Lösung aus 40 g Norbornen, 0,1 g MEHQ und 61,5 g Acrylsäure wurde unter Lufteinleitung (1L/h), Rühren (280 U/min) und Heizen zugetropft, so dass die Innentemperatur 90-92° C betrug. Es wurde 1 h bei Temperatur nachgerührt. Die auf Raumtemperatur abgekühlte Reaktionsmischung wurde mit 300 mL Dichlormethan und mit 200 mL 12,5%iger NaOH-Lösung versetzt und extrahiert. Die Phasen wurden getrennt, die organische Phase nochmals mit 100 mL 12,5%iger NaOH-Lösung extrahiert und nach Phasenseparation konzentriert. Es wurden 71,6 g Produkt (94,5% Ausbeute) in einer Reinheit von 99,5 Gew.% erhalten. Das Produkt wurde mit 7 mg MEHQ stabilisiert. Die Farbzahl betrug 145 Hazen.

### Beispiel 2: Norbornylacrylat unter Vorlage von Dioxan:

In einem 250 mL Vierhalsrundkolben mit Rückflusskühler, Magnetrührer, Thermometer, Tropftrichter und Lufteinleitung wurden 5 mL Dioxan vorgelegt. 0,5 ml Bortrifluoriddimethyletherat wurden zugegeben. Eine Lösung aus 40 g Norbornen, 0,1 g MEHQ und 61,5 g Acrylsäure wurde unter Lufteinleitung (1L/h) unter Rühren (280 U/min) und Heizen zugetropft, so dass die Innentemperatur 90-92° C betrug. Es wurde 1 h bei Temperatur nachgerührt. Die auf Raumtemperatur abgekühlte Reaktionsmischung wurde mit 300 mL Dichlormethan und mit 200 mL 12,5%iger NaOH-Lösung versetzt und extrahiert. Die Phasen wurden getrennt, die organische Phase nochmals mit 100 mL 12,5%iger NaOH-Lösung extrahiert und nach Phasenseparation konzentriert. Es wurden 65,5 g Produkt (92,8% Ausbeute) in einer Reinheit von 99,5 Gew.% erhalten. Das Produkt wurde mit 7 mg MEHQ stabilisiert. Die Farbzahl betrug 74 Hazen.

### Beispiel 3: Norbornylmethacrylat unter Vorlage von Dioxan:

In einem 500 mL Vierhalsrundkolben mit Rückflusskühler, Magnetrührer, Thermometer, Tropftrichter und Lufteinleitung wurden 7,5 mL Dioxan vorgelegt. 0,9 ml Bortrifluoriddimethyletherat wurden zugegeben. Eine Lösung aus 75,1 g Norbornen, 0,22 g MEHQ und 138 g Methacrylsäure wurden unter Lufteinleitung (1L/h), Rühren (500 U/min) und Heizen zugetropft, so dass die Innentemperatur 90-95° C betrug. Es wurde 1 h bei Temperatur nachgerührt. Die auf Raumtemperatur abgekühlte Reaktionsmischung wurde mit 200 mL Dichlormethan und mit 300 mL 12,5%iger NaOH-Lösung versetzt und extrahiert. Die Phasen wurden getrennt, die organische Phase nochmals mit 100 mL 12,5%iger NaOH-Lösung extrahiert und nach Phasenseparation noch zweimal mit je 100 mL Wasser extrahiert, die Phasen jeweils getrennt und die organische Phase konzentriert. Es wurden 136 g Produkt (94,7 % Ausbeute) in einer Reinheit von 99,7 Gew.% erhalten. Das Produkt wurde mit 13,6 mg MEHQ stabilisiert. Die Farbzahl betrug 136 Hazen.

### Beispiel 4: Norbornylmethacrylat unter Vorlage von Norbornylmethacrylat

In einem 500 mL Vierhalsrundkolben mit Rückflusskühler, Magnetrührer, Thermometer, Tropftrichter und Lufteinleitung wurden 5 g Norbornylmethacrylat vorgelegt. 1 ml Bortrifluoriddimethyletherat wird zugegeben. Eine Lösung aus 75,1 g Norbornen, 0,22 g MEHQ und 137,5 g Methacrylsäure wird unter Lufteinleitung (1L/h), Rühren (500 U/min) und Heizen zugetropft, so dass die Innentemperatur 95-97° C betrug. Es wurde 1 h bei Temperatur nachgerührt. Die auf Raumtemperatur abgekühlte Reaktionsmischung wurde mit 200 mL Dichlormethan und mit 300 mL 12,5%iger NaOH-Lösung versetzt und extrahiert. Die Phasen wurden getrennt, die organische Phase nochmals mit 100 mL 12,5%iger NaOH-Lösung extrahiert und nach Phasenseparation noch zweimal mit je 100 mL Wasser extrahiert, die Phasen jeweils getrennt und die organische Phase konzentriert. Es wurden 145 g Produkt (97,6 % Ausbeute) in einer Reinheit von 99,9 Gew.% erhalten. Das Produkt wurde mit 14,6 mg MEHQ stabilisiert. Die Farbzahl betrug 206 Hazen.

### Beispiel 5: Norbornylmethacrylat unter Vorlage von Methacrylsäure:

In einem 2 L Vierhalsrundkolben mit Normagaufsatz, Magnetrührer, Thermometer, Tropftrichter und Lufteinleitung wurden 202 g Methacrylsäure, 0,56 g MEHQ sowie 2,5 mL Bortrifluoriddimethyletherat vorgelegt. Die Mischung wurde unter Rühren (500 U/min) und Lufeinleitung (1L/h) aufgeheizt und eine Lösung von 275 g Norbornen und 100 g Methacrylsäure zugetropft, so dass die Innentemperatur 93-97 ° C betrug.
Nach beendeter Zugabe wurde noch 3,5 h nachgerührt. Überschüssige Säure wurde im Vakuum abdestilliert. Die auf Raumtemperatur abgekühlte Reaktionsmischung wird mit 30%i-ger NaOH extrahiert (105 g) und die Phasen getrennt. Es folgten zwei weitere Extraktionen und Phasenseparationen mit 250 mL Wasser und 50 mL gesättigter Kochsalzlösung sowie 100 mL Wasser und 150 mL gesättigter Kochsalzlösung.
Die organische Phase wurde mit 50 mg MEHQ versetzt, bei 60° C bis 5,5 mbar konzentriert um Spuren von Wasser und ggf. Methacrylsäure zu entfernen und anschließend filtriert. Es wurden 493,4 g Produkt (Ausbeute 93,7 %) mit einer Reinheit von 99,4 Gew.% erhalten. Die Farbzahl betrug 81 Hazen.

### Beispiel 6: Norbornylmethacrylat unter Vorlage von Methacrylsäure:

In einem 500 mL Vierhalsrundkolben mit Rückflusskühler, Magnetrührer, Thermometer, beheizbarem Tropftrichter und Lufteinleitung wurden 54,9 g Methacrylsäure sowie 26 mg MEHQ vorgelegt. 0,65 ml Bortrifluoriddimethyletherat wurden zugegeben. 50 geschmolzenes Norbornen wurde unter Lufteinleitung (1L/h), Rühren (500 U/min) und Heizen zugetropft, so dass die Innentemperatur 85-105° C betrug. Es wurde 5,8 h bei 94° C nachgerührt. Die auf Raumtemperatur abgekühlte Reaktionsmischung wurde mit 200 mL Dichlormethan und mit 20 g 50%iger NaOH-Lösung versetzt und extrahiert. Die Phasen wurden getrennt, die organische Phase, noch zweimal mit je 100 mL 12,5%iger wässriger Kochsalzlösung extrahiert, die Phasen jeweils getrennt und die organische Phase bei 60° C bis 5,5 mbar konzentriert um Spuren von Wasser und ggf. Methacrylsäure zu entfernen. Nach Filtration wurden 92 g Produkt (96,1 % Ausbeute) in einer Reinheit von 99,6 Gew.% erhalten. Die Farbzahl betrug 150 Hazen.

### Beispiel 7: Norbornylmethacrylat unter Vorlage von Methacrylsäure

In einem 2 L Vierhalsrundkolben mit Normagaufsatz, Magnetrührer, Thermometer, Tropftrichter und Lufteinleitung wurden 258 g Methacrylsäure, 0,78 g MEHQ sowie 2,5 mL Bortrifluoriddimethyletherat vorgelegt. Die Mischung wurde unter Rühren (500 U/min) und Lufeinleitung (1L/h) aufgeheizt und eine Lösung von 275 g Norbornen und 245 g Methacrylsäure zugetropft, so dass die Innentemperatur 92-97 ° C betrug.
Nach beendeter Zugabe wurde noch 3 h nachgerührt. Überschüssige Säure wurde teilweise im Vakuum abdestilliert. Die auf Raumtemperatur abgekühlte Reaktionsmischung wurde mit 30%iger NaOH extrahiert (122 g) und die Phasen getrennt. Es folgten zwei weitere Extraktionen und Phasenseparationen mit jeweils 250 mL Wasser und 50 mL gesättigter Kochsalzlösung.
Die organische Phase wurde bei 60° C bis 5,5 mbar konzentriert um Spuren von Wasser und ggf. Methacrylsäure zu entfernen und anschließend filtriert. Es wurden 508,5g Produkt (Ausbeute 96,6%) mit einer Reinheit von 99,8 Gew.% erhalten. Das Endprodukt wurde mit 51 mg MEHQ stabilisiert. Die Farbzahl betrug 39 Hazen.

### Vergleichsbeispiel 1: Norbornylacrylat unter Vorlage von Norbornen:

In einem 250 mL Vierhalsrundkolben mit Rückflusskühler, Magnetrührer, Thermometer, Tropftrichter und Lufteinleitung wurden 40 g Norbornen vorgelegt und aufgeschmolzen. 0,1 g MEHQ und 0,5 ml Bortrifluoriddimethyletherat wurden zugegeben. 61,5 g Acrylsäure wurde bei einer Innentemperatur von anfangs 50° C zugetropft. Nach Beenden des Zutropfens betrug die Innentemperatur 76° C. Die Temperatur wurde auf 90° C erhöht und 5 h nachgerührt. Die auf Raumtemperatur abgekühlte Reaktionsmischung wurde mit 300 mL Dichlormethan und mit 250 mL 8%iger NaHCO3-Lösung versetzt und extrahiert. Es wurden weitere 30 g NaHCO₃ zugegeben. Die Phasen wurden getrennt, die organische Phase nochmals mit 250 mL 8%iger NaHCO₃-Lösung extrahiert und nach Phasenseparation konzentriert. Es wurden 55,3 g Produkt in einer Reinheit von 70,8 Gew.% erhalten.

### Beispiel 8: Norbornylacrylat unter Vorlage von Acrylsäure:

In einem 500 mL Vierhalsrundkolben mit Rückflusskühler, Magnetrührer, Thermometer, beheizbarem Tropftrichter und Lufteinleitung wurden 76,5 g Acrylsäure sowie 52 mg MEHQ vorgelegt. 0,65 ml Bortrifluoriddimethyletherat wurden zugegeben. 50 geschmolzenes Norbornen wurde unter Lufteinleitung (1L/h), Rühren (500 U/min) und Heizen zugetropft, so dass die Innentemperatur 85-100° betrug. Es wurde 5,3 h bei 94° C nachgerührt. Die auf Raumtemperatur abgekühlte Reaktionsmischung wurde mit 20 g 50%iger NaOH-Lösung versetzt und extrahiert. Die Phasen wurden getrennt, die organische Phase, noch zweimal mit je 100 mL 12,5%iger wässriger Kochsalzlösung extrahiert, die Phasen jeweils getrennt und die organische Phase bei 60° C bis 5,5 mbar konzentriert um Spuren von Wasser und ggf. Acrylsäure zu entfernen. Nach Filtration wurden 91 g Produkt (95,1 % Ausbeute) in einer Reinheit von 97 Gew.% erhalten. Die Farbzahl betrug 34 Hazen.

## Patentansprüche

1. Verfahren zur Herstellung von Norbornyl-(meth)acrylat durch Umsetzung von Norbornen mit (Meth)acrylsäure in Gegenwart von Bortrifluorid als Katalysator, **dadurch gekennzeichnet, dass** man
a) Bortrifluorid in (Meth)acrylsäure vorlegt,
b) die Vorlage auf eine Temperatur von 75 bis 110°C erwärmt,
c) Norbornen zugibt
und
d) das erhaltene Norbornyl-(meth)acrylat aus der Reaktionsmischung isoliert.

2. Verfahren zur Herstellung von Norbornyl-(meth)acrylat durch Umsetzung von Norbornen mit (Meth)acrylsäure in Gegenwart von Bortrifluorid als Katalysator, **dadurch gekennzeichnet, dass** man
a) Bortrifluorid in einem organischen Lösungsmittel vorlegt,
b) die Vorlage auf eine Temperatur von 75 bis 110°C erwärmt,
c) eine Mischung enthaltend Norbornen und (Meth)acrylsäure zugibt
und
d) das erhaltene Norbornyl-(meth)acrylat aus der Reaktionsmischung isoliert.

3. Verfahren nach Anspruch 1 oder 2, **dadurch gekennzeichnet, dass** man Bortrifluorid in einer Menge von 0,1 bis 5 Molprozent, bezogen auf die Menge an Norbornen einsetzt.

4. Verfahren nach einem der Ansprüche 1 bis 3, **dadurch gekennzeichnet, dass** man Bortrifluorid in einer Menge von 0,5 bis 1,5 Molprozent, bezogen auf die Menge an Norbornen einsetzt.

5. Verfahren nach einem der Ansprüche 1 bis 4, **dadurch gekennzeichnet, dass** man Bortrifluorid als Diethyletherat, Dimethyletherat oder einer beliebigen Mischung dieser einsetzt.

6. Verfahren nach einem der Ansprüche 2 bis 5, **dadurch gekennzeichnet, dass** es sich bei dem organischen Lösungsmittel um Ether, Norbornyl-(meth)acrylat, (Meth)acrylsäure oder einer beliebigen Mischung dieser handelt.

7. Verfahren nach Anspruch 6, **dadurch gekennzeichnet, dass** es sich bei dem organischen Lösungsmittel um (Meth)acrylsäure handelt.

8. Verfahren nach einem der Ansprüche 1 bis 7, **dadurch gekennzeichnet, dass** die Umsetzung in Gegenwart eines Stabilisators stattfindet und es sich bei dem Stabilisator um Phenotiazin, ein oder mehrere phenolische Verbindungen, ein oder mehrere N-Oxyle oder eine beliebige Mischung dieser handelt.

9. Verfahren nach Anspruch 8, **dadurch gekennzeichnet, dass** der Stabilisator para-Methoxyphenol (MEHQ) ist.

10. Verfahren nach einem der Ansprüche 1 bis 9, **dadurch gekennzeichnet, dass** die Umsetzung in Gegenwart von Sauerstoff stattfindet.

11. Verfahren nach einem der Ansprüche 1 bis 10, **dadurch gekennzeichnet, dass** (Meth)acrylsäure in einer Menge von 100 bis 1000 Molprozent bezogen auf die Menge an Norbornen eingesetzt wird.

12. Verfahren nach einem der Ansprüche 1 bis 11, **dadurch gekennzeichnet, dass** die Isolierung von Norbornyl-(meth)acrylat aus der Reaktionsmischung einen oder mehrere Extraktionsschritte umfasst.

13. Verfahren nach einem der Ansprüche 1 bis 12, **dadurch gekennzeichnet, dass** die Isolierung von Norbornyl-(meth)acrylat aus der Reaktionsmischung die Abtrennung der leichter flüchtigen Verbindungen durch Destillation umfasst.

14. Verfahren nach Anspruch 12 oder 13, **dadurch gekennzeichnet, dass** das so isolierte Norbornyl-(meth)acrylat eine Reinheit von mindestens 99 Gewichtsprozent aufweist.

## Claims

1. A method for preparing norbornyl (meth)acrylate by reacting norbornene with (meth)acrylic acid in the presence of boron trifluoride as catalyst, wherein
a) boron trifluoride is initially charged in (meth)acrylic acid,
b) the initial charge is heated to a temperature of 75 to 110°C,
c) norbornene is added
and
d) the norbornyl (meth)acrylate obtained is isolated from the reaction mixture.

2. A method for preparing norbornyl (meth)acrylate by reacting norbornene with (meth)acrylic acid in the presence of boron trifluoride as catalyst, wherein
a) boron trifluoride is initially charged in an organic solvent,
b) the initial charge is heated to a temperature of 75 to 110°C,
c) a mixture comprising norbornene and (meth)acrylic acid is added
and
d) the norbornyl (meth)acrylate obtained is isolated from the reaction mixture.

3. The method according to claim 1 or 2, wherein boron trifluoride is used in an amount of 0.1 to 5 mole percent, based on the amount of norbornene.

4. The method according to any of claims 1 to 3, wherein boron trifluoride is used in an amount of 0.5 to 1.5 mole percent, based on the amount of norbornene.

5. The method according to any of claims 1 to 4, wherein boron trifluoride is used in the form of the diethyl etherate, dimethyl etherate or any desired mixture of these.

6. The method according to any of claims 2 to 5, wherein the organic solvent is ether, norbornyl (meth)acrylate, (meth)acrylic acid or any desired mixture of these.

7. The method according to claim 6, wherein the organic solvent is (meth)acrylic acid.

8. The method according to any of claims 1 to 7, wherein the reaction takes place in the presence of a stabilizer and the stabilizer is phenothiazine, one or more phenolic compounds, one or more N-oxyls or any desired mixture of these.

9. The method according to claim 8, wherein the stabilizer is para-methoxyphenol (MEHQ).

10. The method according to any of claims 1 to 9, wherein the reaction takes place in the presence of oxygen.

11. The method according to any of claims 1 to 10, wherein (meth)acrylic acid is used in an amount of 100 to 1000 mole percent, based on the amount of norbornene.

12. The method according to any of claims 1 to 11, wherein the isolation of norbornyl (meth)acrylate from the reaction mixture comprises one or more extraction steps.

13. The method according to any of claims 1 to 12, wherein the isolation of norbornyl (meth)acrylate from the reaction mixture comprises separating off the more volatile compounds by distillation.

14. The method according to claim 12 or 13, wherein the norbornyl (meth)acrylate thus isolated has a purity of at least 99 percent by weight.

## Revendications

1. Procédé pour la préparation de (méth)acrylate de norbornyle par transformation de norbornène avec un acide (méth)acrylique en présence de trifluorure de bore en tant que catalyseur, **caractérisé en ce qu'**on
a) charge du trifluorure de bore dans un acide (méth)acrylique,
b) chauffe la charge à une température de 75 à 110 °C,
c) ajoute du norbornène
et
d) isole du mélange réactionnel le (méth)acrylate de norbornyle obtenu.

2. Procédé pour la préparation de (méth)acrylate de norbornyle par transformation de norbornène avec un acide (méth)acrylique en présence de trifluorure de bore en tant que catalyseur, **caractérisé en ce qu'**on
a) charge du trifluorure de bore dans un solvant organique,
b) chauffe la charge à une température de 75 à 110 °C,
c) ajoute un mélange contenant du norbornène et un acide (méth)acrylique
et
d) isole du mélange réactionnel le (méth)acrylate de norbornyle obtenu.

3. Procédé selon la revendication 1 ou 2, **caractérisé en ce qu'**on utilise le trifluorure de bore en une quantité de 0,1 à 5 pour cent en moles, par rapport à la quantité de norbornène.

4. Procédé selon l'une quelconque des revendications 1 à 3, **caractérisé en ce qu'**on utilise le trifluorure de bore en une quantité de 0,5 à 1,5 pour cent en moles, par rapport à la quantité de norbornène.

5. Procédé selon l'une quelconque des revendications 1 à 4, **caractérisé en ce qu'**on utilise le trifluorure de bore en tant que diéthyléthérate, diméthyléthérate ou en tant qu'un quelconque mélange de ceux-ci.

6. Procédé selon l'une quelconque des revendications 2 à 5, **caractérisé en ce que** le solvant organique est un éther, un (méth)acrylate de norbornyle, un acide (méth)acrylique ou un quelconque mélange de ceux-ci.

7. Procédé selon la revendication 6, **caractérisé en ce que** le solvant organique est un acide (méth)acrylique.

8. Procédé selon l'une quelconque des revendications 1 à 7, **caractérisé en ce que** la transformation a lieu en présence d'un stabilisant et le stabilisant est la phénothiazine, un ou plusieurs composés phénoliques, un ou plusieurs N-oxyles ou un quelconque mélange de ceux-ci.

9. Procédé selon la revendication 8, **caractérisé en ce que** le stabilisant est le para-méthoxyphénol (MEHQ).

10. Procédé selon l'une quelconque des revendications 1 à 9, **caractérisé en ce que** la transformation a lieu en présence d'oxygène.

11. Procédé selon l'une quelconque des revendications 1 à 10, **caractérisé en ce que** l'acide (méth)acrylique est utilisé en une quantité de 100 à 1 000 pour cent en moles par rapport à la quantité de norbornène.

12. Procédé selon l'une quelconque des revendications 1 à 11, **caractérisé en ce que** l'isolement du (méth)acrylate de norbornyle du mélange réactionnel comprend une ou plusieurs étapes d'extraction.

13. Procédé selon l'une quelconque des revendications 1 à 12, **caractérisé en ce que** l'isolement du (méth)acrylate de norbornyle du mélange réactionnel comprend la séparation des composés plus volatils par distillation.

14. Procédé selon la revendication 12 ou 13, **caractérisé en ce que** le (méth)acrylate de norbornyle ainsi isolé présente une pureté d'au moins 99 pour cent en poids.
